# EUROPÄISCHE PATENTSCHRIFT

(11) **EP 0 758 551 B1**
(45) Veröffentlichungstag und Bekanntmachung des Hinweises auf die Patenterteilung: **01.12.1999**
(21) Anmeldenummer: 96110340.5
(22) Anmeldetag: 27.06.1996
(51) Int. Cl.: A61M 1/00, B01D 29/03, A61C 8/00, A61B 17/58

(54) **Sammelvorrichtung**
Collection device
Dispositif de collection

(30) Priorität: 16.08.1995 DE 29513090 U
(43) Veröffentlichungstag der Anmeldung: 19.02.1997
(73) Patentinhaber: Friadent GmbH, 68222 Mannheim (DE)
(72) Erfinder: Haessler, Dieter Dr., D-55276 Oppenheim (DE); Zöller, J. PD Dr. Dr., D-69126 Heidelberg (DE)
(74) Vertreter: Klose, Hans, Dipl.-Phys.

(56) Entgegenhaltungen:
- DE-A- 3 045 245
- DE-A- 4 300 116
- DE-U- 9 418 004
- DE-U- 9 420 694
- US-A- 4 083 706
- US-A- 4 393 879
- US-A- 5 108 381

## Beschreibung

Die Erfindung bezieht sich auf eine Sammelvorrichtung für Knochenmaterial in der Medizintechnik gemäß den im Oberbegriff des Patentanspruchs 1 angegebenen Merkmalen. Eine derartige Sammelvorrichtung ist aus der US-A-4083706 bekannt.

Die Insertion von ausreichend dimensionierten enossalen Implantaten ist im wesentlichen vom Knochenangebot abhängig. Deshalb wurden in den letzten Jahren unterschiedliche Externsions- und Augmentationsplastiken entwickelt, um den Alveolarfortsatz zu rekonstruieren und ein dauerhaft belastungsfähiges Implantatlager zu schaffen. In Abhängigkeit von der Defektgröße kommen Auffüllungen mit Knochenersatzmaterialien wie beispielsweise Hydroxylapatit, oder autogene, homogene sowie heterogene Knochentransplantate zur Anwendung. Aufgrund seiner osteoplastischen Potenz bei fehlenden immunologischen Reaktionen stellt autogener Knochen das Transplantationsmaterial der ersten Wahl dar. Als wesentlicher Nachteil mußte bisher in Kauf genommen werden, daß zur Gewinnung regelmäßig ein Zweiteingriff notwendig ist und infolgedessen Beeinträchtigungen des Spenderareals auftreten.

Hiervon ausgehend liegt der Erfindung die Aufgabe zugrunde, eine Sammelvorrichtung für Knochenmehl oder Knochenspäne vorzuschlagen, welche insbesondere bei der Präparation des Implantatbetts anfallen. Die Vorrichtung soll einen funktionssicheren Aufbau besitzen und eine einfache, insbesondere intraoperative, Handhabung ermöglichen, und eine einfache und schnelle Entleerung des gewonnenen Knochenmaterials ermöglichen.

Die Lösung dieser Aufgabe erfolgt gemäß den Merkmalen des Patentanspruchs 1.

Die vorgeschlagene Sammelvorrichtung ermöglicht problemlos das Sammeln des bei der Präparation des Implantatlagers, einer Kieferkammglättung oder dergleichen anfallenden Knochenmaterials, welches oftmals auch als Knochenmehl oder Knochenspäne bezeichnet wird. Anstelle der bisher üblichen Beseitigung des Knochenmaterials beim Absaugen der Kühlflüssigkeit wird jenes nunmehr mittels der Sammelvorrichtung aufgesaugt und gesammelt. Die Gewinnung von autogenem Knochenmaterial und die Preparation des Implantatbettes erfolgt in einem einzigen chirurgischen Eingriff. Eine zusätzliche Belastung des Patienten infolge einer Eröffnung eines Donorfeldes wird vermieden. Die Sammelvorrichtung enthält eine Sieb, insbesondere aus Titan oder einem vergleichbaren Werkstoff, welches bedarfsweise aus der Vorrichtung herausnehmbar ist. Das Sieb enthält vorzugsweise Poren, im wesentlichen mit einer Größe zwischen 100 bis 1200 µm, vorzugsweise zwischen 200 und 1000 µm, und ermöglicht somit das Auffangen und Zurückhalten des anfallenden Knochenmaterials.

Die Poren weisen in besonders zweckmäßiger Weise eine Länge auf, welche um einen vorgegebenen Faktor größer ist als die Breite. Bevorzugt ist die Länge der Poren wenigstens um den Faktor 2 bis 3 größer als die Breite der Poren. Durch Ausbildung der Poren als Schlitze mit einer größeren Länge als Breite wird in vorteilhafter Weise die Ausbeute des gewonnenen autogenen Materials erhöht, ohne daß ein Zusetzen der Poren zu befürchten wäre. Die Querschnitts länge der Poren liegt in zweckmäßiger Weise zwischen 200 bis 1000 µm, wobei sich ein Verhältnis der Breite zur Länge zwischen 1:2 bis 1:3 als besonders zweckmäßig erwiesen hat.

Das Sieb ist bevorzugt als eine im wesentlichen plane Scheibe und ohne Rand ausgebildet, so daß das Abtragen der gesammelten Knochensubstanz problemlos, insbesondere mittels Spatel, erfolgen kann. Im Unterschied zu topfförmigen ausgebildeten Sieben gewährleistet die randfreie Siebausbildung eine vollständige Weiterverwendung der gesammelten Knochenspäne und eine sichere Reinigung und Sterilisation.

Die Sammelvorrichtung gelangt vor allem in der Dentalmedizin zur Verwendung und ist ferner problemlos zerlegbar, und zwar vorzugsweise ohne zusätzliche Werkzeuge, so daß auch intraoperativ eine einfache und schnelle Entleerung des gewonnenen Knochenmaterials ermöglicht wird. Die Vorrichtung besitzt einen Anschluß, insbesondere in Form eines Stutzens für eine Absaugleitung bzw. Saugkanüle, zwecks Vorgabe eines Unterdrucks zum Absaugen. Der Anschluß ist vorzugsweise als einfacher Steckanschluß ausgebildet, so daß problemlos und/oder ohne zusätzliche Werkzeuge die Verbindung mit der Absaugleitung hergestellt oder gelöst werden kann, um somit eine einfache Desinfektion und/oder Sterilisation der Sammelvorrichtung zu ermöglichen.

Weiterbildungen und besondere Ausgestaltungen der Erfindung sind in den Unteransprüchen angegeben.

Anhand der Zeichnung wird eine besondere Ausführungsform der Erfindung erläutert, ohne daß hierbei eine Beschränkung allein auf diese Ausführungsform erfolgt. Es zeigen:
- Fig. 1: die Sammelvorrichtung teilweise in einer seitlichen Ansicht und teilweise in einem Axialschnitt,
- Fig. 2: eine Aufsicht auf das im wesentlichen plan ausgebildete Sieb.

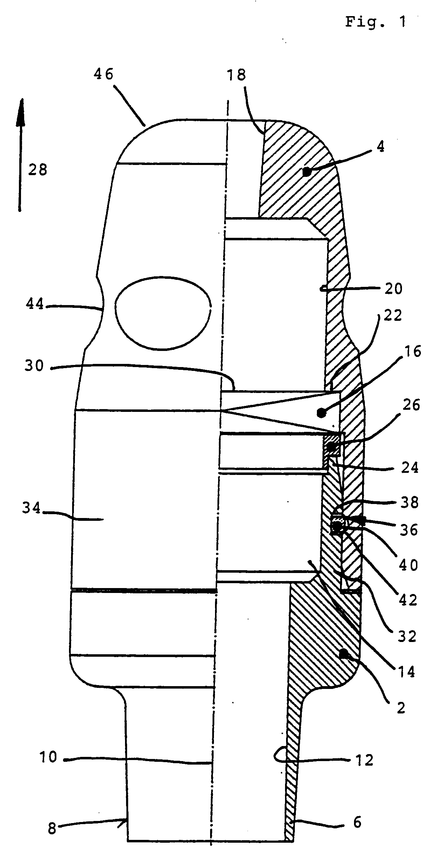

In der Fig. 1 ist teilweise in einer Ansicht und teilweise in einem Axialschnitt die Sammelvorrichtung etwa im Maßstab 10:1 vergrößert dargestellt, welche ein Gehäuse 2 und eine mit diesem verbundene Kappe 4 aufweist. Das Gehäuse 2 weist an seinem hinteren, freien Ende einen Anschluß 6 für eine Absaugleitung oder Saugkanüle auf. Der Anschluß 6 ist in zweckmäßiger Weise als Stutzen ausgebildet, auf dessen Außenfläche 8 die schlauchartig ausgebildete Absaugleitung aufgeschoben werden kann. Wie ersichtlich, kann die Absaugleitung problemlos abgezogen werden, um die Reinigung und/oder Sterilisation bei Bedarf durchführen zu können. Zum einfachen Aufstecken der Absaugleitung ist die Außenfläche 8 zweckmäßig leicht konisch ausgebildet, wobei die Konusspitze auf der anderen Seite als die Kappe 4 bezüglich des Gehäuses 2 liegt, und zwar insbesondere auf der Längsachse 10.

Die insbesondere zur Verwendung in der Dentalmedizin ausgebildete Sammelvorrichtung ist im Inneren hohl ausgebildet, wobei das Gehäuse 2 innen eine vorzugsweise axiale Durchgangsbohrung 12 enthält. Im vorderen Bereich des Gehäuses 2 enthält die Durchgangsbohrung 12 eine Erweiterung. Im Innenraum der Sammelvorrichtung ist ferner ein Sieb 16 angeordnet, welches insbesondere als ein Titanblech ausgebildet ist. Das Sieb oder Filter 16 besitzt eine vorgegebene Porengröße, welche insbesondere im Bereich zwischen 100 bis 1200 µm, vorzugsweise 200 bis 1000 µm, liegt und das Sammeln des Knochenmaterials in der benötigten Größe ermöglicht. Zweckmäßig enthält das Sieb längliche Poren, so daß einerseits die Ausbeute am gewonnenen autologen Knochenmaterial erhöht wird und andererseits ein unerwünschtes Zusetzen vermieden wird. Bei den vorzugsweise schlitzförmig ausgebildeten Poren hat sich ein Verhältnis von Breite : Länge im Bereich zwischen 1:2 bis 1:3 als zweckmäßig erwiesen.

Die Kappe 4 enthält innen eine Durchgangsbohrung mit einem vorderen engen Bereich 18 und einem erweiterten inneren Bereich 20. Aufgrund der Durchmesserdifferenz wird somit die Saugwirkung beim Ansaugen verbessert. Im Anschluß an den erweiterten Bereich 20 besitzt die Kappe 4 innen einen Ringbund 22, an welchem das Sieb 16 mit seinem äußeren Rand anliegt. Am inneren Ende 24 des Gehäuses 2 ist ein federelastischer Ring 26, insbesondere aus Elastomer oder Gummi, vorgesehen. Dieser elastische Ring 26 liegt einerseits am inneren Ende 24 des Gehäuses und andererseits am Sieb 16 an. Wie ersichtlich, ist somit das Sieb 16 zwischen dem Gehäuse 2 und der Kappe 4 festgelegt und definiert eingespannt. Die Kappe 4 kann in Richtung des Pfeiles 28 nach vorn vom Gehäuse 2 abgzogen werden, wobei das Sieb 16 freigelegt und an dessen Vorderseite 30 angesammeltes Knochenmaterial entfernt werden kann. Der Ring 26 dient ferner auch als Abdichtung, so daß bei der Meidung von Fremdluft eine einwandfreie Absaugung sichergestellt ist. Die Innendurchmesser der Erweiterung 14 im Gehäuse 2 sowie des erweiterten Bereiches 20 der Durchgangsbohrung der Kappe 4 sind zweckmäßigerweise im wesentlichen gleich groß ausgebildet.

Die Kappe 4 übergreift mit ihrem hinteren Ende 34 den vorderen Teil 32 des Gehäuses 2, wobei Verbindungselemente 36 vorgesehen sind. Die Verbindungselemente 36 sind in zweckmäßger Weise als eine Rast- oder Schnappverbindung ausgebildet, um eine einfache und schnelle Entkopplung der Kappe 4 vom Gehäuse 2 und letztendlich eine einfache und schnelle Entleerung des mittels des Siebs 16 gewonnen Knochenmaterials zu ermöglichen. Das Gehäuse 2 enthält hierbei in einer äußeren Ringnut 38 einen elastisch nachgiebigen Ring 40, welcher insbesondere als O-Ring ausgebildet ist. Korrespondierend weist die Kappe 4 im hinteren Ende 32 innen eine Ringnut 42 auf, in welche der elastische Ring 40 teilweise eingreift. Die Ringnuten 38 und 42 sowie der elastische Ring 40 sind derart aufeinander abgestimmt, daß nach Überwindung einer vorgebbaren Haltekraft die Kappe 4 in Richtung des Pfeiles 28 vom Gehäuse 2 abgezogen werden kann.

Obgleich die erläuterte Rast- oder Schnappverbindung einen geringen Herstellungsaufwand erfordert und gleichwohl eine funktionssichere Verbindung bei einfacher Lösbarkeit gewährleistet, können im Rahmen der Erfindung auch andere Verbindungsmittel vorgesehen sein. Nur beispielshaft sei an dieser Stelle auf eine Gewindeverbindung oder auf einen Bajonettverschluß hingewiesen. Zur Erleichterung der Handhabung weist die Kappe 4 außenliegende Rillen oder Grifflächen 44 auf, welche als Vertiefungen in der Außenfläche der Kappe 4 ausgebildet sind und das problemlose Ergreifen der Kappe mit den Fingern ermöglichen. Die Kappe 4 ist an der Spitze 46 abgerundet, um Verletzungen zu vermeiden.

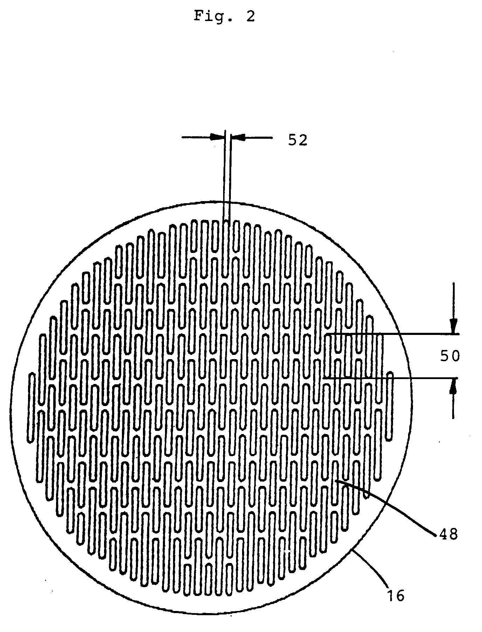

Fig. 2 zeigt eine axiale Ansicht des Siebes 16, welches als eine plane Scheibe ausgebildet ist. Ein in Achsrichtung über die Ebene der Scheibe vorstehender Rand ist nicht vorhanden. Das aus Titan bestehende Sieb 16 enthält eine Anzahl von Poren, welche als längliche Schlitze 48 ausgebildet sind. Die Poren 48 sind parallel zueinander ausgerichtet und besitzen eine Länge 50 sowie eine Breite 52. Wesentlich ist, daß die Länge 50 um einen vorgegebenen Faktor größer ist als die Breite 52. Der Faktor ist zweckmäßig größer als zwei und vorzugsweise größer als drei. Die Länge 50 liegt im Bereich zwischen 400 bis 1200 µm. Als untere Grenze für die Breite 52 haben sich 100 µm als zweckmäßig erwiesen, doch kann im Rahmen der Erfindung als untere Grenze für die Breite 52 auch 200 µm angesetzt werden. Aufgrund der erfindungsgemäß vorgegebenen länglichen Ausbildung der Poren bzw. Schlitze werden die Knochenspäne optimal gefiltert, ohne daß ein Zusetzen der Poren zu befürchten wäre.

Wie aus Fig. 2 ersichtlich, sind im Randbereich des Siebes 16 relativ große Schlitze vorhanden, so daß gerade im Randbereich ein zu schnelles Zusetzen der Poren verhindert wird. Ferner wird durch die somit im Vergleich zu den übrigen Stegen in der Länge vergrößert ausgebildeten Stegen zwischen den Poren oder Schlitze die Stabilität und Festigkeit des Siebes verbessert.

Bei dem in Fig. 2 dargestellten Sieb 16 liegen die Längskanten der einzelnen Poren ebenso wie die Poren selbst parallel zueinander. Alternativ können im Rahmen der Erfindung die Poren 48 auch abweichende Querschnittsformen aufweisen, wobei hier vor allem auf ovale Querschnitte verwiesen sei. Unabhängig von der konkreten Formgebung der einzelnen Poren kommt es maßgeblich darauf an, daß die Querschnittsachse in der einen Richtung um einen vorgegebenen Faktor größer ist als die Achse in der hierzu orthogonalen Richtung.

### Bezugszeichen

- 2: Gehäuse
- 4: Kappe
- 6: Anschluß / Stutzen
- 8: Außenfläche von 6
- 10: Längsachse
- 12: Durchgangsbohrung
- 14: Erweiterung
- 16: Sieb
- 18: vorderer Bereich in 4
- 20: erweiterter Bereich in 4
- 22: Ringbund
- 24: Inneres Ende von 2
- 26: Elastischer Ring
- 28: Pfeil
- 30: Vorderseite von 16
- 32: vorderer Teil von 2
- 34: hinteres Ende von 4
- 36: Verbindungselement
- 38: Ringnut in 32
- 40: Ring
- 42: Innere Ringnut in 34
- 44: Grifffläche
- 46: Spitze von 4
- 48: Pore
- 50: Länge
- 52: Breite

## Patentansprüche

1. Sammelvorrichtung für Knochenmaterial in der Medizintechnik, enthaltend ein Gehäuse (2) mit einem Anschluß (6) für eine Saugleitung, einen mit dem Gehäuse (2) verbundenen und von diesem abnehmbaren Halter sowie im Inneren der Sammelvorrichtung ein zwischen dem Gehäuse (2) und dem Halter festgelegtes Sieb (16), welches durch Zerlegen der Sammelvorrichtung zugänglich ist, wobei das Gehäuse (2) und der Hafer eine Durchgangsbohrung aufweisen,
dadurch gekennzeichnet,
daß der Halter als eine Kappe (4) ausgebildet ist, daß im Inneren der Kappe (4) distal von dem Sieb (16) ein erweiterter Bereich (20) angeordnet ist, in welchem Knochenmaterial ansammelbar ist, daß die Kappe (4) im Bereich einer Spitze (46) einen vorderen Bereich (18) der Durchgangsbohrung aufweist, wobei der Durchmesser des vorderen Bereiches (18) kleiner ist als der Durchmesser des im Inneren der Kappe (4) angeordneten erweiterten Bereiches (20), und daß das Sieb (16) an einem inneren Ende (24) des Gehäuses (2) anliegt, so daß nach dem Abnehmen der Kappe (4) das am distalen Ende des Siebes (16) angesammelte Knochenmaterial ohne Beeinträchtigung durch das Gehäuse (2) entfernbar ist.

2. Sammelvorrichtung nach Anspruch 1, dadurch gekennzeichnet, daß die Kappe (4) einen Ringbund (22) oder dergleichen aufweist, an welchem das Sieb (16), vorzugsweise mit einem äußeren Rand, anliegt.

3. Sammelvorrichtung nach Anspruch 2, dadurch gekennzeichnet, daß zwischen dem Gehäuse (2) und der Kappe (4) Verbindungselemente (36) angeordnet sind, welche insbesondere durch in Richtung einer Längsachse (10) wirkende und in der Größe vorgebbare Zugkräfte eine Trennung der Kappe (4) vom Gehäuse (2) ermöglichen, wobei die Verbindungselemente bevorzugt einen nachgiebigen Ring (40) aufweisen, welcher teilweise in eine Ringnut (38) des Gehäuses und teilweise in eine korrespondierende Ringnut (42) der Kappe (4) eingreift.

4. Sammelvorrichtung nach einem der Ansprüche 1 bis 3, dadurch gekennzeichnet, daß zusammen mit dem axial zwischen dem Gehäuse (2) und der Kappe (4) festgelegten Sieb (16) ein elastischer Ring (26) vorgesehen ist.

5. Sammelvorrichtung nach eincm der Ansprüche 1 bis 4; dadurch gekennzeichnet, daß das Sieb (16) Poren (48) aufweist, deren Querschnittslänge (50) um einen vorgegebenen Faktor größer ist als deren Breite (52) und daß dieser Faktor bevorzugt einen Wert 2 oder größer besitzt.

6. Sammelvorrichtung nach Anspruch 5, dadurch gekennzeichnet, daß die Poren (48) oder deren großen Längsachsen im wesentlichen parallel zueinander angeordnet sind, wobei das Sieb (16) bevorzugt als eine im wesentlichen plane Scheibe ausgebildet ist.

7. Sammelvorrichtung nach einem der Ansprüche 1 bis 6, dadurch gekennzeichnet, daß die Poren einen Durchmesser oder eine Querschnittslänge im Bereich zwischen 100 bis 1200 µm, zweckmäßig zwischen 200 und 1000 µm aufweisen.

8. Sammelvorrichtung nach einem der Ansprüche 1 bis 7, dadurch gekennzeichnet, daß zwischen dem inneren Ende (24) des Gehäuses (2) und dem Sieb (16) ein elastischer Ring (26) angeordnet ist.

9. Sammelvorrichtung nach einem der Ansprüche 1 bis 8, dadurch gekennzeichnet, daß die Kappe (4) mit einem hinteren Ende (34) einen vorderen Teil (32) des Gehäuses (2) übergreift, wobei zwischen dem hinteren Ende (34) und dem vorderen Teil (32) vorzugsweise die Verbindungselemente (36) angeordnet sind.

## Claims

1. Collecting device for bone material in medicine, comprising a housing (2) with a connection (6) for a suction line, a holder which is connected to the housing (2) and can be removed from this and, inside the collecting device, a screen (16) which is fixed between the housing (2) and the holder and is accessible by dismantling the collecting device, the housing (2) and the holder having a through bore,
characterized in that
the holder is constructed as a cap (4), in that a widened region (20) in which bone material can be collected is arranged inside the cap (4) distally of the screen (16), in that the cap (4) has, in the region of a tip (46), a front region (18) of the through bore, the diameter of the front region (18) being smaller than the diameter of the widened region (20) arranged inside the cap (4), and in that the screen (16) lies against an inner end (24) of the housing (2), so that after the cap (4) has been removed, the bone material collected at the distal end of the screen (16) can be removed without obstruction by the housing (2).

2. Collecting device according to claim 1, characterized in that the cap (4) has an annular collar (22) or the like, against which the screen (16) lies, preferably with an outer edge.

3. Collecting device according to claim 2, characterized in that between the housing (2) and the cap (4) are arranged connecting elements (36) which allow separation of the cap (4) from the housing (2), in particular by tensile forces which act in the direction of a longitudinal axis (10) and can be preset in size, the connecting elements preferably having a flexible ring (40), which engages partly in an annular groove (38) of the housing and partly in a corresponding annular groove (42) of the cap (4).

4. Collecting device according to one of claims 1 to 3, characterized in that an elastic ring (26) is provided together with the screen (16) fixed axially between the housing (2) and the cap (4).

5. Collecting device according to one of claims 1 to 4, characterized in that the screen (16) has pores (48), the cross-sectional length (50) of which is greater than the width (52) thereof by a given factor, and in that this factor preferably has a value of 2 or more.

6. Collecting device according to claim 5, characterized in that the pores (48) or long longitudinal axes thereof are arranged substantially parallel to one another, the screen (16) preferably being constructed as a substantially flat disc.

7. Collecting device according to one of claims 1 to 6, characterized in that the pores have a diameter or a cross-sectional length in the range between 100 to 1,200 µm, expediently between 200 and 1,000 µm.

8. Collecting device according to one of claims 1 to 7, characterized in that an elastic ring (26) is arranged between the inside end (24) of the housing (2) and the screen (16).

9. Collecting device according to one of claims 1 to 8 characterized in that the cap (4) overlaps with a rear end (34) a front part (32) of the housing (2), the connecting elements (36) preferably being arranged between the rear end (34) and the front part (32).

## Revendications

1. Dispositif de prélèvement de matériau osseux en technique médicale, comprenant un boîtier (2) avec un raccord (6) pour une conduite d'aspiration, un support relié au boîtier (2) et séparable de celui-ci, ainsi que, à l'intérieur du dispositif de prélèvement, un tamis (16) immobilisé entre le boîtier (2) et le support, qui est accessible par démontage du dispositif de prélèvement, le boîtier (2) et le support présentant un passage,
***caractérisé en ce que*** le support est conformé en capuchon (4), ***en ce qu***'à l'intérieur du capuchon (4), de manière distale par rapport au tamis (16), est placée une zone élargie (20), dans laquelle du matériau osseux peut s'accumuler, ***en ce que*** le capuchon (4) présente au niveau d'une pointe (46) une zone avant (18) du passage, le diamètre de la zone avant (18) étant inférieur au diamètre de la zone élargie (20) placée à l'intérieur du capuchon (4), et ***en ce que*** le tamis (16) s'appuie contre une extrémité intérieure (24) du boîtier (2), de façon qu'après le retrait du capuchon (4), le matériau osseux accumulé à l'extrémité distale du tamis (16) puisse être retiré sans gêne du boîtier (2).

2. Dispositif de prélèvement selon la Revendication 1, ***caractérisé en ce que*** le capuchon (4) présente une collerette (22) ou analogue, contre laquelle s'appuie le tamis (16), de préférence avec un bord extérieur.

3. Dispositif de prélèvement selon la Revendication 2, ***caractérisé en ce qu***'entre le boîtier (2) et le capuchon (4) sont placés des éléments de liaison (36), qui en particulier, par le biais de force de traction agissant dans la direction d'un axe longitudinal (10) et d'amplitude prédéterminable, permettent de séparer le capuchon (4) du boîtier (2), les éléments de liaison présentant de préférence une bague souple (40), laquelle s'engage partiellement dans une rainure annulaire (38) du boîtier et partiellement dans une rainure annulaire correspondante (42) du capuchon (4).

4. Dispositif de prélèvement selon l'une quelconque des Revendications 1 à 3, ***caractérisé en ce qu***'avec le tamis (16) immobilisé axialement entre le boîtier (2) et le capuchon (4), il est prévu une bague élastique (26).

5. Dispositif de prélèvement selon l'une quelconque des Revendications 1 à 4, ***caractérisé en ce que*** le tamis (16) présente des pores (48), dont la longueur de section (50) est supérieure d'un facteur prédéterminé à leur largeur (52) et ***en ce que*** ce facteur possède de préférence une valeur de 2 ou plus.

6. Dispositif de prélèvement selon la Revendication 5, ***caractérisé en ce que*** les pores (48) ou leurs grands axes sont disposés sensiblement parallèles les uns aux autres, le tamis (16) étant conformé de préférence en disque pour l'essentiel plan.

7. Dispositif de prélèvement selon l'une quelconque des Revendications 1 à 6, ***caractérisé en ce que*** les pores présentent un diamètre ou une longueur de section compris entre 100 et 1200 µm, de préférence entre 200 et 1000 µm.

8. Dispositif de prélèvement selon l'une quelconque des Revendications 1 à 7, ***caractérisé*** ***en ce qu***'entre l'extrémité intérieure (24) du boîtier (2) et le tamis (16) est placée une bague élastique (26).

9. Dispositif de prélèvement selon l'une quelconque des Revendications 1 à 8, ***caractérisé en ce que*** le capuchon (4) saisit, avec une extrémité arrière (34), une partie avant (32) du boîtier (2), les éléments de liaison (36) étant placés de préférence entre l'extrémité arrière (34) et la partie avant (32).
